# EUROPÄISCHE PATENTANMELDUNG

(11) **EP 3 992 121 A1**
(43) Veröffentlichungstag der Anmeldung: **04.05.2022**
(21) Anmeldenummer: 21204519.9
(22) Anmeldetag: 25.10.2021
(51) Int. Cl.: B65G 51/26

(54) **VERFAHREN ZUM STEUERN EINER ROHRPOSTANLAGE**

(30) Priorität: 23.10.2020 AT 509172020
(71) Anmelder: Ing. Sumetzberger GmbH, 1110 Wien (AT)
(72) Erfinder: Friedrich, Peter, 1040 Wien (AT)
(74) Vertreter: Müllner, Martin

(57) **Zusammenfassung**

Bei einem Verfahren zum Steuern einer Rohrpostanlage, wobei Rohrposthülsen von verschiedenen Absendestationen zu zumindest einer Zentralstation gesendet werden, wo die Rohrposthülsen automatisch entladen werden, wobei sie danach zu derselben oder einer anderen Absendestation zurückgeschickt werden, soll eine automatische Desinfektion der Rohrposthülsen integriert werden, ohne Gefahr, dass der Inhalt der Rohrposthülsen beschädigt wird. Erfindungsgemäß werden die Rohrposthülsen beim Rücktransport von der Zentralstation zunächst durch eine Desinfektionsstation geleitet, wo sie chemisch und/oder durch UV-Strahlung desinfiziert werden, und erst danach zu der Absendestation weitergeleitet. Auf diese Weise werden nur leere Rohrposthülsen desinfiziert. Wenn zumindest einige Rohrposthülsen auch bei der Sendung von der Absendestation zur Zentralstation durch die Desinfektionsstation geleitet werden, bleibt die Desinfektionsstation vorzugsweise abgeschaltet. Zusätzlich können leere Rohrposthülsen manuell zur Desinfektionsstation geschickt werden.

## Beschreibung

### Technisches Gebiet

Die vorliegende Erfindung betrifft ein Verfahren zum Steuern einer Rohrpostanlage, wobei Rohrposthülsen von verschiedenen Absendestationen zu zumindest einer Zentralstation gesendet werden, wo die Rohrposthülsen automatisch entladen werden, wobei sie danach zu derselben oder einer anderen Absendestation zurückgeschickt werden.

### Stand der Technik

Solch ein Verfahren ist z.B. in EP 2463662 A beschrieben. Wie in dieser Schrift dargelegt, werden in modernen Krankenhausanlagen immer häufiger zentrale Labors installiert, um Kosten, Ressourcen und Zeit zu sparen. Von mehreren Stellen eines Krankenhauses (Notaufnahme, Operationssaal, Pathologie etc.) werden Blutproben, Gewebeproben und sonstige zu analysierende Proben in ein zentrales Labor gesendet, wo sie dann analysiert werden. Für Transportzwecke müssen daher solche Krankenhaus-Rohrpostanlagen groß dimensioniert werden, d.h. es kommen viele Stationen und sehr viele Rohrposthülsen zum Einsatz.

Aus diesem Grund ist bei derartigen Rohrpostanlagen vorgesehen, dass die Rohrposthülsen beim Labor (oder bei den Labors), also der Zentralstation (oder den Zentralstationen) automatisch entleert werden. Im einfachsten Fall kann zu diesem Zweck die Rohrposthülse unten geöffnet werden, und die Proberöhrchen mit den Blutproben fallen in einen Auffangtrichter der Analysestation. Anschließend wird automatisch kontrolliert (z.B. mit einem optischen Sensor), ob tatsächlich alle Proberöhrchen herausgefallen sind, und die leere Rohrposthülse wird entweder zum Absender oder auch zu einer anderen Station gesendet; letzteres insbesondere dann, wenn bei dieser anderen Station gerade zu wenige Rohrposthülsen vorhanden sind, beim Absender aber noch genug Rohrposthülsen zur Verfügung stehen.

Dabei ist problematisch, dass die Rohrposthülsen mit Keimen (Bakterien, Viren) infiziert sein können. Bei der Zentralstation ist das wenig problematisch, weil dort die Rohrposthülsen automatisch entleert werden, also im Normalbetrieb mit keinem Menschen in Kontakt kommen. Allerdings werden die Rohrposthülsen wieder zu den absendenden Stationen zurückgeschickt, wo sie in der Folge neuerlich mit Proberöhrchen beladen werden, und das geschieht in den meisten Fällen händisch. D.h. wenn die Rohrposthülse infiziert ist, kann sich die Person, die die Rohrposthülse mit Proberöhrchen belädt, selbst infizieren.

Das wird insbesondere dann problematisch, wenn die Rohrposthülsen nicht immer zur selben Absendestation zurückgeschickt werden; dann kann sich eine Infektion über die Rohrpostanlage durch ein gesamtes Krankenhaus verbreiten.

Nun ist auch schon vorgeschlagen worden, Rohrposthülsen zu desinfizieren. Das kann händisch erfolgen, was klarerweise arbeitsintensiv ist; es wurden aber auch schon Desinfektionsstationen vorgeschlagen, wo die Desinfektion automatisch erfolgt, sei es chemisch, indem die Rohrposthülse mit einem Desinfektionsmittel besprüht wird, sei es durch Bestrahlung mittels UV-Licht.

Problematisch bei solchen Desinfektionsstationen ist, dass man nicht jede Hülse automatisch desinfizieren kann, denn z.B. Blutproben sind empfindlich gegen UV-Licht, und Rohrposthülsen haben meist ein transparentes Hülsenrohr, damit man den Inhalt sieht. Wenn also eine Rohrposthülse, die mit Blutproben beladen ist, in solch einer Desinfektionsstation desinfiziert wird, werden die Blutproben unbrauchbar.

Es ist daher derzeit üblich, leere Rohrposthülsen bei Bedarf händisch zu einer Desinfektionsstation zu schicken. Die Erfahrung hat aber gezeigt, dass das insbesondere an "hektischen" Tagen, also an Tagen mit hoher Arbeitsbelastung, oft vernachlässigt wird, aber gerade an solch "hektischen" Tagen besonders viele Rohrposthülsen verschickt werden, sich also eine Infektion besonders schnell über die Rohrpostanlage verbreiten kann.

### Kurzbeschreibung der Erfindung

Es ist Aufgabe der vorliegenden Erfindung, hier Abhilfe zu schaffen.

Diese Aufgabe wird durch ein Verfahren der eingangs genannten Art erfindungsgemäß dadurch gelöst, dass jede Rohrposthülse beim Rücktransport von der Zentralstation zunächst durch eine Desinfektionsstation geleitet wird, wo sie chemisch und/oder durch UV-Strahlung desinfiziert wird, und erst danach zu der Absendestation weitergeleitet wird.

Der vorliegenden Erfindung liegt die - im Nachhinein betrachtet sehr simple - Erkenntnis zu Grunde, dass bei einem Verfahren der eingangs genannten Art bekannt ist, wann Rohrposthülsen voll und wann sie leer sind: Rohrposthülsen, die zur Zentralstation unterwegs sind, sind voll, und Rohrposthülsen, die von der Zentralstation kommen, sind leer. Daher können alle Rohrposthülsen, die von der Zentralstation kommen, gefahrlos desinfiziert werden, und bei den Absendestationen kommen nur desinfizierte Rohrposthülsen an. Bei der Zentralstation könnten zwar infizierte Rohrposthülsen ankommen, aber wie schon erwähnt kommen die Rohrposthülsen dort im Regelfall nicht mit Menschen in Kontakt.

Aus technischen Gründen kann es notwendig sein, dass zumindest einige Rohrposthülsen auch bei der Sendung von der Absendestation zur Zentralstation durch die Desinfektionsstation geleitet werden, nämlich dann, wenn keine Umgehungsleitung um die Desinfektionsstation vorhanden ist und auch kein anderer Weg zur Verfügung steht. In diesem Fall ist nach einer Ausgestaltung der Erfindung vorgesehen, dass die Desinfektionsstation abgeschaltet bleibt, wenn solch eine Rohrposthülse die Desinfektionsstation durchfährt.

Schließlich kann auch vorgesehen sein, dass zusätzlich leere Rohrposthülsen manuell zur Desinfektionsstation geschickt werden können, wie dies bisher praktiziert wird.

### Kurze Beschreibung der Zeichnungsfiguren

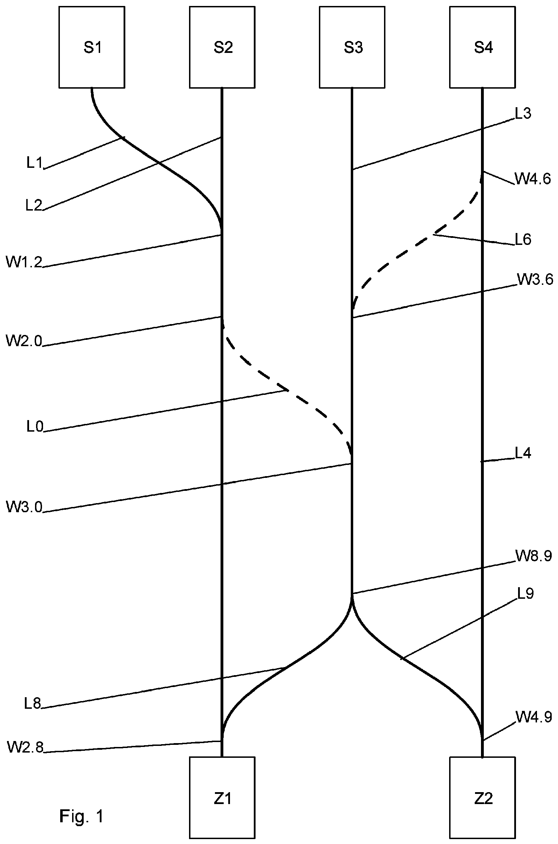

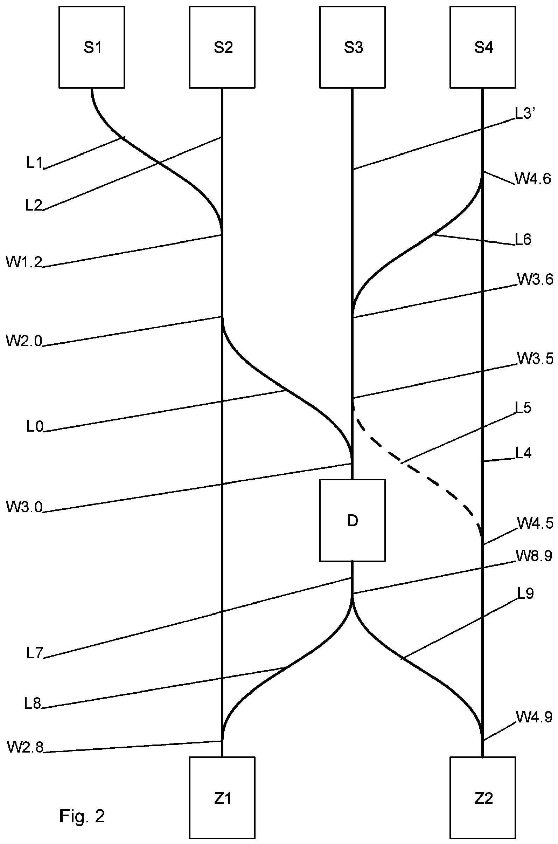

Anhand der beiliegenden Zeichnung wird die vorliegende Erfindung näher erläutert. Es zeigt: Fig. 1 schematisch eine herkömmliche Rohrpostanlage und Fig. 2 dieselbe Rohrpostanlage, bei der eine Desinfektionsstation eingebaut ist, sodass das erfindungsgemäße Verfahren implementierbar ist.

### Beschreibung der Ausführungsarten

Gemäß Fig. 1 sind 4 Absendestationen S1, S2, S3 und S4 sowie zwei Zentralstationen Z1 und Z2 vorgesehen. Die Absendestation S2 ist über eine gerade Rohrleitung L2 mit der Zentralstation Z1 verbunden. Über eine Weiche W1.2 führt eine (Abzweig-)Rohrleitung L1 zur Absendestation S1. Somit können beide Absendestationen S1 und S2 Rohrposthülsen über die Leitung L2 direkt zur Zentralstation Z1 schicken.

Die Absendestation S4 ist über eine gerade Rohrleitung L4 mit der Zentralstation Z2 verbunden. Somit kann die Absendestation S4 Rohrposthülsen über die Leitung L4 direkt zur Zentralstation Z2 schicken.

Die Absendestation S3 ist an einer Rohrleitung L3 angeschlossen. Von dieser Rohrleitung L3 führen über eine Weiche W8.9 zwei Rohrleitungen L8 und L9 zu den Rohrleitungen L2 und L4, wo sie über Weichen W2.8 und W4.9 zusammengeführt werden. Die Absendestation S3 kann somit Rohrposthülsen sowohl zur Zentralstation Z1 als auch zur Zentralstation Z2 schicken.

Damit die Anlage flexibler wird, sieht man weiter (Verbindungs-)Rohrleitungen L0 und L6 vor (in Fig. 1 strichliert eingezeichnet). Die Rohrleitung L0 verbindet über Weichen W2.0 und W3.0 die Rohrleitung L2 mit der Rohrleitung L3, wogegen die Rohrleitung L6 über Weichen W4.6 und W 3.6 die Rohrleitung L4 mit der Rohrleitung L3 verbindet. Es ist unmittelbar ersichtlich, dass nun jede Absendestation S1, S2, S3 und S4 Rohrposthülsen zu beiden Zentralstationen Z1 und Z2 schicken kann.

Solch eine Rohrpostanlage lässt sich leicht mit einer Desinfektionsstation D erweitern, wie dies in Fig. 2 dargestellt ist: Die Rohrleitung L3 wird unterbrochen, sodass zwei Rohrleitungen L3' und L7 entstehen, und dazwischen wird die Desinfektionsstation D angeordnet. Man erkennt unmittelbar, dass beide Zentralstationen Z1 und Z2 Rohrposthülsen über die Desinfektionsstation D an jede Absendestation S1, S2, S3 und S4 schicken können, sodass beim Rücktransport alle Rohrposthülsen desinfiziert werden können. Allerdings muss bei dieser Konfiguration die Absendestation S3 alle weggesendeten Rohrposthülsen über die Desinfektionsstation D schicken. Auch wenn die Desinfektionsstation D dabei abgeschaltet bleibt, ist dies insofern nachteilig, als die Absendestation S3 keine Rohrposthülsen wegsenden kann, solange Rohrposthülsen in der Desinfektionsstation D desinfiziert werden. Es ist daher zweckmäßig, eine weitere (Verbindungs-)Rohrleitung L5 (strichliert eingezeichnet) vorzusehen, die die Rohrleitung L3 über Weichen W3.5 und W4.5 mit der Rohrleitung L4 verbindet. Nun können die Absendestationen S1 und S2 Rohrposthülsen direkt (unter Umgehung der Desinfektionsstation D) zur Zentralstation Z1 schicken, und die Absendestationen S3 und S4 können Rohrposthülsen direkt (unter Umgehung der Desinfektionsstation D) zur Zentralstation Z2 schicken.

Die Desinfektionsstation D muss nur dann mit vollen Rohrposthülsen durchfahren werden, wenn die Absendestationen S1 und S2 Rohrposthülsen zur Zentralstation Z2 schicken möchten oder wenn die Absendestationen S3 und S4 Rohrposthülsen zur Zentralstation Z1 schicken möchten.

## Patentansprüche

1. Verfahren zum Steuern einer Rohrpostanlage, wobei Rohrposthülsen von verschiedenen Absendestationen zu zumindest einer Zentralstation gesendet werden, wo die Rohrposthülsen automatisch entladen werden, wobei sie danach zu derselben oder einer anderen Absendestation zurückgeschickt werden, **dadurch gekennzeichnet, dass** jede Rohrposthülse beim Rücktransport von der Zentralstation zunächst durch eine Desinfektionsstation geleitet wird, wo sie chemisch und/oder durch UV-Strahlung desinfiziert wird, und erst danach zu der Absendestation weitergeleitet wird.

2. Verfahren nach Anspruch 1, **dadurch gekennzeichnet, dass** zumindest einige Rohrposthülsen auch bei der Sendung von der Absendestation zur Zentralstation durch die Desinfektionsstation geleitet werden, wobei aber die Desinfektionsstation abgeschaltet bleibt.

3. Verfahren nach Anspruch 1 oder 2, **dadurch gekennzeichnet, dass** zusätzlich leere Rohrposthülsen manuell zur Desinfektionsstation geschickt werden.
